# EUROPÄISCHE PATENTSCHRIFT

(11) **EP 2 358 657 B1**
(45) Veröffentlichungstag und Bekanntmachung des Hinweises auf die Patenterteilung: **17.07.2013**
(21) Anmeldenummer: 09756482.7
(22) Anmeldetag: 19.11.2009
(51) Int. Cl.: C07C 67/08, C07C 69/54

(54) **VERFAHREN ZUR HERSTELLUNG VON METHACRYLIERTEN BENZOPHENONEN**
METHOD FOR PRODUCING METHACRYLATED BENZOPHENONES
PROCÉDÉ DE FABRICATION DE BENZOPHÉNONES MÉTHACRYLÉES

(30) Priorität: 15.12.2008 DE 102008054611
(43) Veröffentlichungstag der Anmeldung: 24.08.2011
(73) Patentinhaber: Evonik Röhm GmbH, 64293 Darmstadt (DE)
(72) Erfinder: KNEBEL, Joachim, 64665 Alsbach-Hähnlein (DE); KLESSE, Wolfgang, 55127 Mainz (DE); BREINER, Christine Maria, 69514 Laudenbach (DE); SCHMITT, Gerold, 63743 Aschaffenburg (DE)
(86) Internationale Anmeldenummer: PCT/EP2009/065431
(87) Internationale Veröffentlichungsnummer: WO 2010/072479

(56) Entgegenhaltungen:
- EP-A1- 0 869 138
- EP-A1- 1 676 870
- EP-A2- 0 246 848
- EP-A2- 0 487 293
- WO-A1-03/033452
- US-A- 3 214 492
- US-A- 3 265 772
- US-A- 3 315 013
- ZENJIRO OSAWA ET AL: "Preparation of Ultraviolet Stabilizing Polymers. I. Copolymerization of 2-Hydroxy-4-acryloyloxybenzophenone and Its Ultraviolet Stability" JOURNAL OF MACROMOLECULAR SCIENCE : PART A - CHEMISTRY, MARCEL DEKKER, NEW YORK, NY, US, Bd. 1, Nr. 4, 1. Juli 1967 (1967-07-01), Seiten 581-590, XP008119113 ISSN: 0022-233X
- NEIDLINGER H H ET AL: "Effect of polymeric 2-hydroxybenzophenone stabilizers on the weathering of PMMA films" POLYMER PREPRINTS, AMERICAN CHEMICAL SOCIETY, US, Bd. 28, Nr. 1, 1. Januar 1987 (1987-01-01), Seiten 205-206, XP008119138 ISSN: 0032-3934
- TOOMEY R ET AL: "Swelling behavior of thin, surface-attached polymer networks" MACROMOLECULES, Bd. 37, Nr. 3, 2004, Seiten 882-887, XP002570741
- DATABASE WPI Week 200437 Thomson Scientific, London, GB; AN 2004-393757 XP002570753 & JP 2003 261506 A (MITSUBISHI RAYON CO LTD) 19. September 2003 (2003-09-19) in der Anmeldung erwähnt
- VRETIK L ET AL: "Polymethacryloylaminoarylmethacrylates: New Concept of Photoalignment Materials for Liquid Crystals" MOLECULAR CRYSTALS AND LIQUID CRYSTALS, Bd. 479, 1. Januar 2007 (2007-01-01), XP008095216 ISSN: 1542-1406

## Beschreibung

Die Erfindung betrifft ein Verfahren zur Herstellung von methacrylierten Benzophenonen und deren Verwendungen.

Im Stand der Technik ist ein Methacrylsäureanhydridverfahren zur Herstellung der oben genannten Ester beschrieben (JP2003261506, Mitsubishi Rayon). Als Katalysator wird Triethylamin eingesetzt. Da das Amin mit der bei der Reaktion entstehenden Methacrylsäure ein Salz bildet, muß es äquimolar zum Benzophenon bemessen werden. Entsprechend erhält man äquimolare Salzmengen, die als Abfall entsorgt werden müssen. Das Verfahren ist von daher wenig wirtschaftlich.

Weitere Methoden des Standes der Technik sind die Umsetzung von Methacrylsäurechlorid mit hydroxyfunktionellen Benzophenon sowie die Umsetzung dieses Rohstoffs mit Glycidylmethacrylat. Beim Umgang mit Methacrylsäurechlorid müssen die korrosiven und ätzenden Eigenschaften beachtet werden. Bei Kontakt mit Wasser wird zudem HCl frei.

In der DE 1720603 wird ein Verfahren zur Herstellung von wässrigen Dispersionen leicht vernetzbarer Polymerisate beschrieben. Hierbei werden Acryl- und Methacrylsäureester mit lichtaktiven, olefinisch ungesättigten Monomeren copolymerisiert , ggf. unter Mitverwendung von lichtaktiven, nichtionogenen Emulgatoren.

Die EP0346788 beschreibt ein Verfahren zur Herstellung strahlungsempfindlicher Carbamoylbenzo- und -acetophenone mit mindestens einer Methacrylat- bzw. Acrylatendgruppe. Dabei werden Isocyanatoalkyl(meth)acrylate mit Hydroxyacetophenonen oder Hydroxybenzophenonen mit einem basischen Katalysator umgesetzt. Hierbei muß unter Feuchtigkeitsausschluß gearbeitet werden. Zudem können nur getrocknete nicht nucleophile Lösungsmittel verwendet werden.

Aufgabe war es, ein verbessertes Verfahren zur Herstellung von (Meth)acrylsäureestern von hydroxyfunktionellen Benzophenonen zur Verfügung zu stellen.

Die Aufgabe wurde gelöst durch ein Verfahren zur Herstellung von Benzophenon(meth)acrylaten, dadurch gekennzeichnet, dass Hydroxybenzophenone und (Meth)acrylsäureanhydrid in Gegenwart katalytischer Säuremengen umgesetzt werden, dann der Katalysator neutralisiert wird und anschließend das Rohmonomer aufgereinigt wird.

Die Schreibweise (Meth)acrylat bedeutet hier sowohl Methacrylat, wie z.B. Methylmethacrylat, Ethylmethacrylat usw., als auch Acrylat, wie z.B. Methylacrylat, Ethylacrylat usw., sowie Mischungen aus beiden.
Überraschend wurde gefunden, dass mit dem erfindungsgemäßen Verfahren hohe Umsätze erzielt werden, und die Menge der Nebenprodukte stark reduziert wird.
Es wurde gefunden, dass das erfindungsgemäße Verfahren nur durch eine geringe Salzfracht belastet ist, die entsteht, wenn die Katalysatorsäure bei der Aufarbeitung neutralisiert wird. Die als Nebenprodukt anfallende Methacrylsäure kann bei der anschließenden Polymerisation des Benzophenonmonomeren als Comonomer mit verwendet werden oder zur Herstellung von neuem Methacrylsäureanhydrid recycliert werden.
Die Umsetzung kann in Gegenwart gängiger Alkyl- oder Arylsulfonsäuren, bevorzugt mit Schwefelsäure erfolgen.
Vorzugsweise werden 4-Hydroxybenzophenon und (Meth)acrylsäureanhydrid in Gegenwart katalytischer Mengen konzentrierter Schwefelsäure umgesetzt.
(Meth)acrylsäureanhydrid wird in leichtem Überschuß zum Hydroxybenzophenon gegeben. Die Umsetzung erfolgt bei Temperaturen zwischen 50 bis 120°C, vorzugsweise bei 80°C bis 100°C, über 4 bis 8 Stunden, bevorzugt über 5,5 bis 6,5 Stunden.
Die Neutralisation der katalytisch eingesetzten Säure erfolgt mit wässrigen Basen, vorzugsweise mit Alkalilauge oder Ammoniaklösung.

Die anschließende Aufbereitung des Rohmonomers erfolgt durch Zugabe von Wasser. So werden die Verunreinigungen gelöst und können problemlos abgetrennt werden. Die wasserlöslichen Verunreinigungen der [(Meth)acryloyloxy)benzophenon-Schmelze werden vorzugsweise durch die Zugabe von Wasser entfernt.
[(Meth)acryloyloxy]benzophenon wird durch die Zugabe überschüssigen Wassers in das Reaktionsgemisch ausgefällt und durch Filtration in fester Form isoliert.
Die in hoher Reinheit hergestellten Benzophenon(meth)acrylate können in Lösung mit Methylmethacrylat, n-Butylmethacrylat, i-Butylmethacrylat oder Styrol gelagert und weiter umgesetzt werden.

Benzophenon(meth)acrylate können zur nachträglichen Photovernetzung von Polymeren durch Tages- oder UV-licht sowie als polymere Photoinitiatoren verwendet werden.
Die Benzophenon(meth)acrylate können zudem als Comonomer für Polymerisationsreaktionen verwendet werden.
Die im Folgenden gegebenen Beispiele werden zur besseren Veranschaulichung der vorliegenden Erfindung gegeben, sind jedoch nicht dazu geeignet, die Erfindung auf die hierin offenbarten Merkmale zu beschränken.

### BEISPIELE

### Beispiel 1:

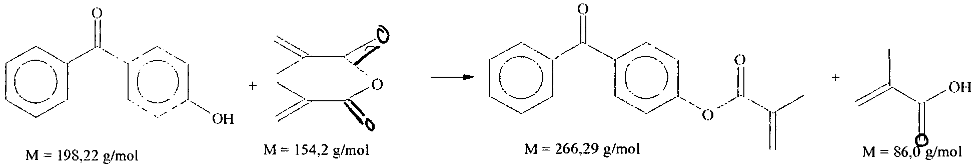

Apparatur: 4 l Vierhalsrundkolben mit mechanischem Rührer, Rückflusskühler, Pt100-Temperaturfühler, Lufteinleitrohr, Anschützaufsatz, Tropftrichter, elektrisch beheiztem Ölbad

### Ansatz:

3,5 mol 4-Hydroxybenzophenon, 99,7% : 695,9 g
3,85 mol Methacrylsäureanhydrid (stabilisiert mit 2000 ppm 2,4-Dimethyl-6-tert-butylphenol) : 618,4 g
0,020 mol konzentrierte Schwefelsäure : 1,99 g
1864 mg Hydrochinonmonomethylether
932 mg 2,4-Dimethyl-6-tert-butylphenol

Neutralisation der Katalysatorsäure mit 1,8 g Natronlauge gelöst in 10 g Wasser Veresterung des überschüssigen Methacrylsäureanhydrids mit 22,4 g Methanol
Theoretische Ausbeute: 930,0 g

### Durchführung:

Der Ansatz wurde komplett eingewogen und dann unter Rühren und Einleiten von Luft auf 90°C erhitzt. Reaktionszeit bei 90°C: 6h. Es wurde dann auf ca. 60°C abgekühlt und das in Wasser gelöste Natriumhydroxid zur Neutralisation der Katalysatorschwefelsäure sowie das Methanol zur Veresterung des unumgesetzten Methacrylsäureanhydrids zugegeben. Anschließend wurde 1 h bei 60°C gerührt, dann wurde der Ansatz unter Rühren (Metallflügelrührer, Rührmotor) im dünnen Strahl in 3 l Wasser gegossen. Es wurde 0,5 h gerührt und die Ausfällung dann über eine Glasfilterfritte abgesaugt, noch zweimal mit je 2 l Wasser nachgewaschen; und anschließend auf der Nutsche mittels Luft vorgetrocknet. Der Feststoff wurde anschließend an der Luft getrocknet.

| | |
|---|---|
| Ausbeute: | 924,6 g (99,4 % d. Theorie) |
| Analysen: | Wassergehalt: 0,08% |
| | Hydrochinonmonomethylether: 6 ppm |
| | 2,4-Dimethyl-6-tert-butylphenol: 174 ppm |

Gaschromatographie:
0,047 % Methylmethacrylat
0,013 % Methacrylsäure
0,637 % 4-Hydroxybenzophenon
97,56% 4-(Methacryloyloxy)benzophenon
Pt-Co-Farbzahl als 20%ige Lösung in Aceton: 150

### Beispiel 2:

Apparatur: 4 l Vierhalsrundkolben mit mechanischem Rührer, Rückflusskühler, Pt100-Temperaturfühler, Lufteinleitrohr, Anschützaufsatz, Tropftrichter, elektrisch beheiztem Ölbad

### Ansatz:

1,5 mol 4-Hydroxybenzophenon : 303 g
1,65 mol Methacrylsäureanhydrid (stabilisiert mit 2000 ppm 2,4-Dimethyl-6-tert-butylphenol) : 262 g
0,0087 mol konzentrierte Schwefelsäure : 0,84 g
798 mg Hydrochinonmonomethylether
399 mg 2,4-Dimethyl-6-tert-butylphenol

### Durchführung:

Der Ansatz wurde komplett eingewogen und dann unter Rühren und Einleiten von Luft auf 90°C erhitzt. Reaktionszeit bei 90°C: 6h. Es wurde dann auf ca. 60°C abgekühlt und das in Wasser gelöste Natriumhydroxid zur Neutralisation der Katalysatorschwefelsäure sowie das Methanol zur Veresterung des unumgesetzten Methacrylsäureanhydrids zugegeben. Anschließend wurde 1 h bei 60°C gerührt, dann wurde der Ansatz unter Rühren mit 1566 g Methylmethacrylat versetzt. Die entstandene Lösung wurde unter Rühren auf Raumtemperatur gekühlt und filtriert. Die Lösung des 4-(Methacryloyloxy)benzophenons in Methylmethacrylat hat die folgende gaschromatographisch bestimmte Zusammensetzung:

| | |
|---|---|
| 56,016 % | Methylmethacrylat |
| 6,954 % | Methacrylsäure |
| 2,399 % | 4-Hydroxybenzophenon |
| 32,717 % | 4-(Methacryloyloxy)benzophenon. |

Der Wassergehalt beträgt 0,27 %, der Stabilisatorgehalt 113 ppm 2,4-Dimethyl-6-tert-butylphenol und 4 ppm Hydrochinonmonomethylether. Die Pt-Co_Farbzahl ist 169.

## Patentansprüche

1. Verfahren zur Herstellung von Benzophenon(meth)acrylaten, **dadurch gekennzeichnet, dass** Hydroxybenzophenone und (Meth)acrylsäureanhydrid in Gegenwart katalytischer Mengen konzentrierter Schwefelsäure, einer Alkyl-oder Arylsulfonsäure umgesetzt werden, dann der Katalysator neutralisiert wird und anschließend das Rohmonomer aufgereinigt wird.

2. Verfahren gemäß Anspruch 1, **dadurch gekennzeichnet, dass** 4-Hydroxybenzophenon und (Meth)acrylsäureanhydrid in Gegenwart katalytischer Säuremengen umgesetzt werden, die dann mit wässrigen Basen neutralisiert werden und anschließend die löslichen Verunreinigungen der [(Meth)acryloyloxy]benzophenon- Schmelze durch Zugabe von Wasser gelöst werden.

3. Verfahren gemäß Anspruch 1, **dadurch gekennzeichnet, dass** mit wässriger Alkalilauge oder Ammoniaklösung neutralisiert wird.

4. Verfahren gemäß Anspruch 1, **dadurch gekennzeichnet, dass** die Umsetzung über 4-8 Stunden bei 50 bis 120°C erfolgt.

5. Verfahren gemäß Anspruch 1, **dadurch gekennzeichnet, dass** das [(Meth)acryloyloxy]benzophenon durch Zugabe überschüssigen Wassers in das Reaktionsgemisch ausgefällt und durch Filtration in fester Form isoliert wird.

6. Verfahren gemäß Anspruch 1, **dadurch gekennzeichnet, dass** das [(Meth)acryloyloxy]benzophenon durch Zugabe eines flüssigen (Meth)acrylsäureesters oder Styrol in das Reaktionsgemisch als Lösung in diesem Ester verwendet wird.

## Claims

1. Process for preparing benzophenone (meth)acrylates, **characterized in that** hydroxybenzophenones and (meth)acrylic anhydride are reacted in the presence of catalytic amounts of concentrated sulphuric acid, of an alkylsulphonic or arylsulphonic acid, then the catalyst is neutralized, and subsequently the crude monomer is purified.

2. Process according to Claim 1, **characterized in that** 4-hydroxybenzophenone and (meth)acrylic anhydride are reacted in the presence of catalytic amounts of acid, which are then neutralized with aqueous bases, and subsequently the soluble impurities in the [(meth)acryloyloxy]benzophenone melt are dissolved by addition of water.

3. Process according to Claim 1, **characterized in that** neutralization is carried out with aqueous alkali metal hydroxide solution or ammonia solution.

4. Process according to Claim 1, **characterized in that** the reaction takes place over 4-8 hours at 50 to 120°C.

5. Process according to Claim 1, **characterized in that** the [(meth)acryloyloxy]benzophenone is precipitated by addition of excess water to the reaction mixture and is isolated in solid form by filtration.

6. Process according to Claim 1, **characterized in that** the [(meth)acryloyloxy]benzophenone, as a result of addition of a liquid (meth)acrylic ester or styrene into the reaction mixture, is used as a solution **in that** ester.

## Revendications

1. Procédé pour la préparation de (méth)acrylates de benzophénone, **caractérisé en ce qu'**on fait réagir des hydroxybenzophénones et de l'anhydride (méth)acrylique en présence de quantités catalytiques d'acide sulfurique concentré, d'un acide alkyl- ou arylsulfonique, puis on neutralise le catalyseur et ensuite on purifie le monomère brut.

2. Procédé selon la revendication 1, **caractérisé en ce qu'**on fait réagir de la 4-hydroxybenzophénone et de l'anhydride (méth)acrylique en présence de quantités catalytiques d'acide, qui sont ensuite neutralisées avec des bases aqueuses et ensuite on dissout par addition d'eau les impuretés solubles de la masse fondue de [(méth)acryloyloxy]benzophénone.

3. Procédé selon la revendication 1, **caractérisé en ce qu'**on effectue la neutralisation avec une solution aqueuse d'hydroxyde de métal alcalin ou une solution aqueuse d'ammoniac.

4. Procédé selon la revendication 1, **caractérisé en ce que** la réaction s'effectue en l'espace de 4-8 heures à une température de 50 à 120°C.

5. Procédé selon la revendication 1, **caractérisé en ce qu'**on isole sous forme solide la [(méth)acryloyloxy]benzophénone par introduction d'eau en excès dans le mélange réactionnel et par filtration.

6. Procédé selon la revendication 1, **caractérisé en ce que** par introduction d'un ester d'acide (méth)acrylique liquide ou de styrène dans le mélange réactionnel on utilise la [(méth)acryloyloxy]-benzophénone sous forme de solution dans cet ester.
